# EUROPEAN PATENT APPLICATION

(11) **EP 4 797 277 A1**
(43) Date of publication of application: **26.08.2026**
(21) Application number: 26159148.1
(22) Date of filing: 17.02.2026
(51) Int. Cl.: G16H 30/40, G16H 40/67, G16H 50/20, G16H 50/30, G16H 50/70, G16H 80/00, A61B 5/00, A61B 5/16

(54) **CONTINUOUS HEALTH MONITORING SYSTEM VIA VIDEO CONFERENCING STREAMS**

(30) Priority: 24.02.2025 US 202519061918
(71) Applicant: Mitel Networks Corporation, Ottawa, Ontario K2K 3K1 (CA)
(72) Inventor: Murthy, Tejas, 56008 Bangalore (IN); Radhakrishnan, Jayachandran, 560035 Bengaluru (IN); Naidoo, Logendra, Ottawa, K1S 0W8 (CA)
(74) Representative: McDougall, James

(57) **Abstract**

Systems and methods including one or more processors and one or more non-transitory storage devices storing computing instructions configured to run on the one or more processors and perform acts of receiving videoconferencing data of the user from a videoconferencing software; determining one or more multimodal diagnostic features from the videoconferencing data; analyzing the one or more multimodal diagnostic features to determine one or more target datapoints; storing the one or more target datapoints in a data registering apparatus; determining a cumulative severity of health from the one or more target datapoints and the plurality of health datapoints; determining a health anomaly has occurred based on a comparison of the cumulative severity to a predetermined threshold; based on the determined health anomaly, recompiling the one or more target datapoints to confirm the health anomaly; and sending the health anomaly to an external device.

## Description

### TECHNICAL FIELD

This disclosure relates generally to videoconferencing methods and systems and more particularly to methods and systems for detecting health anomalies of a user from videoconferencing data.

### BACKGROUND

Existing methods of monitoring a person's health may include wearable devices, self-reporting, and/or medical checkups. Current health monitoring that relies on self-reporting or infrequent medical check-ups often fail to capture real-time data.

Existing solutions that include wearable devices may track the person's physical activities and vital signs. Medical software applications may also analyze user-entered data to flag potential health issues. In some cases, video conferencing platforms monitor user engagement and fatigue through basic camera and audio analysis and suggest breaks for the user. However, these methods miss real-time, in-situ monitoring, leading to delayed detection and intervention. As a result, the person's productivity declines when health issues are not addressed timely.

Therefore, there is a need for improved methods and systems to timely detect and analyze health issues without interaction from the user or additional devices.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the detailed description and claims when considered in connection with the drawing figures, wherein like numerals denote like elements and wherein:
FIG. 1 is a block diagram of a system according to aspects of this disclosure;
FIG. 2 is a block diagram of a method for detecting health anomalies from videoconferencing data of a user according to aspects of this disclosure;
FIG. 3 is a flowchart for detecting health anomalies from videoconferencing data of a user according to aspects of this disclosure; and
FIG. 4 illustrates a representative block diagram of a computer system, according to an embodiment.

It will be appreciated that elements in the figures are illustrated for simplicity and clarity and have not necessarily been drawn to scale. For example, the dimensions of some of the elements in the figures may be exaggerated relative to other elements to help to improve understanding of illustrated embodiments of the present invention.

### DETAILED DESCRIPTION

The description of exemplary embodiments of the present invention provided herein is merely exemplary and is intended for purposes of illustration only; the following description is not intended to limit the scope of the invention as claimed. Moreover, recitation of multiple embodiments having stated features is not intended to exclude other embodiments having additional features or other embodiments incorporating different combinations of the stated features.

It must also be noted that, the term "exemplary" is used in the sense of "example," rather than "ideal."

It must also be noted that, as used in the specification and the appended claims, the singular forms "a," "an" and "the" include plural referents unless the context clearly dictates otherwise.

By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

Relative terms, such as "about," "substantially," or "approximately" are used to include small variations with specific numerical values (e.g., +/- x%,), as well as including the situation of no variation (+/-0%). In various embodiments, the numerical value x is less than or equal to 10 - e.g., less than or equal to 5, to 2, to 1, or smaller.

As used herein, "database" refers to any suitable database for storing information, electronic files or code to be utilized to practice embodiments of this disclosure. As used herein, "server" refers to any suitable server, computer or computing device for performing functions utilized to practice embodiments of this disclosure.

As used herein, "software" refers to programs or other operating information utilized by a processor or other computing hardware.

As used herein, "meeting" means a meeting or conference such as telephonic, video, audio/video, in-person, a hybrid of any of the preceding, and any type of meeting involving multiple participants.

This disclosure provides a system for detecting health anomalies from videoconferencing of a user. In particular, the system may detect health anomalies from videoconferencing audio or visual data. The system may detect the health anomalies while a videoconferencing software (also referred to herein as "communication apparatus," "video conferencing platform," or "collaboration platform") is in use. The videoconferencing software may be a communication apparatus, such as platforms like Zoom^{™}, Microsoft Teams^{™}, WebEx^{™}, and other similar applications. Leveraging artificial intelligence (AI) driven features, the system can continuously monitor a user's state of health while using the communication apparatus, allowing the system to perform real-time analysis of the user's health data. The system described herein can systematize the capture and analysis of data captured during video conferencing. Then, AI models trained to recognize health-related anomalies can process this data. The system can flag deviations from normal patterns and store timestamped incidents. Health data can then be compiled into a timeline, accessible by authorized health professionals.

Currently, working professionals, among other individuals, often engage in long hours of video conferencing, leading to overlooked health indicators. Significant health issues like stress, diabetes, or early signs of chronic diseases may remain undetected. Current methods of monitoring health data, such as wearable devices, self-reporting, and/or medical checkups, miss real-time, in-situ monitoring, leading to delayed detection and intervention.

Unlike existing methods of health monitoring, the system described herein can utilize continuous audio-visual streams from collaboration platforms to monitor health signs. Therefore, the present system can remedy the above-described deficiencies of existing solutions by providing immediate, ongoing analysis without requiring user interaction or additional devices.

The present systems and methods can integrate artificial intelligence (AI)-driven health monitoring into video conferencing platforms. The AI can continuously the videoconferencing data to detect early signs of health issues. This may allow for real-time health monitoring and immediate flagging of potential health concerns, enhancing early intervention and ongoing health management.

The system described herein can utilize continuous audio-visual streams and other videoconferencing data from collaboration platforms for health monitoring, a capability not present in existing solutions. Traditional methods lack real-time, in-situ analysis, resulting in delayed detection and intervention. The present disclosure can perform constant, automatic analysis (e.g., ongoing analysis of data without any action on the part of the user), eliminating the need for user input or supplementary devices. For individuals engaged in daily, prolonged video or call sessions over an extended period of time, substantial health data accumulates. By utilizing software algorithms, this system can capture and evaluate sensor data (e.g., from a camera, microphone, and the like), alerting users to any detected anomalies. The system described herein can also facilitate connections with health professionals, aligning with the emerging trend of on-demand healthcare.

Given the increasing amount of time people spend in video conferences, this system can leverage large-scale data collection from the video conferences. Regular video conferencing provides continuous, real-time data, making it an effective vehicle for ongoing health monitoring and early medical diagnoses. The integration of the present system into the everyday activity of video conferencing can enable comprehensive health insights without disrupting normal workflows of the user participating in the video conference. Therefore, the system described herein can be an opportunistic tool for preventive healthcare.

The detection AI in the present system may operate by capturing and analyzing video and audio data during video conferences. AI models, specifically trained to identify various health-related anomalies, may continuously process this streaming data. These trained AI models can analyze this data in real-time, using large datasets that represent various health conditions. As some examples, for glaucoma, the AI might analyze changes in the eye's appearance, such as subtle shifts in shape or how light reflects off the pupil, which could be early indicators. For diabetes, the AI may monitor changes in skin color, particularly around the eyes and mouth, which can indicate high blood sugar levels over time, and any visible weight fluctuations. For general anomalies, the AI may assess deviations from established patterns of a user's physical and vocal characteristics, which could signal stress, fatigue, or other health issues.

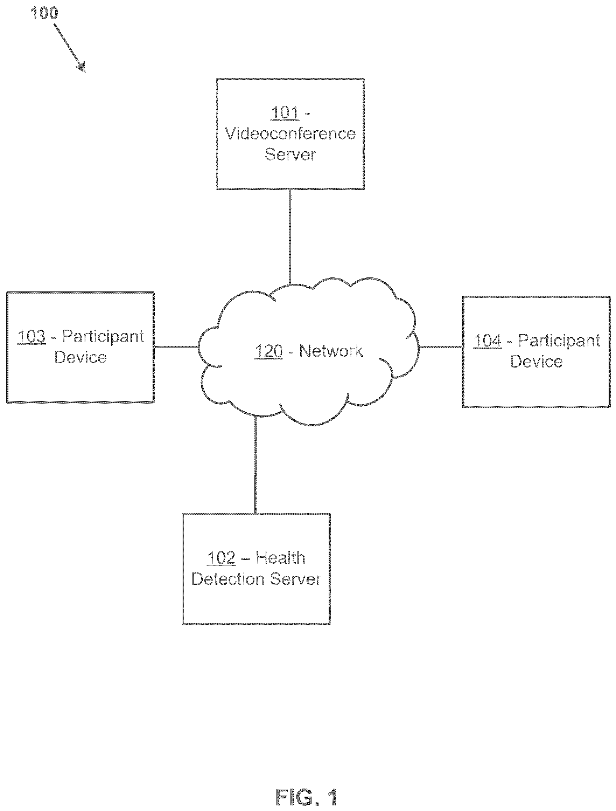

Turning to the figures, FIG. 1 illustrates a block diagram of a system 100 that can be employed for detecting health anomalies, as described in greater detail below. System 100 is merely exemplary and embodiments of the system are not limited to the embodiments presented herein. System 100 can be employed in many different embodiments or examples not specifically depicted or described herein. In some embodiments, certain elements or modules of system 100 can perform various procedures, processes, and/or activities. In these or other embodiments, the procedures, processes, and/or activities can be performed by other suitable elements or modules of system 100.

Generally speaking, system 100 can be implemented with hardware and/or software. Part or all of the hardware and/or software implemented in system 100 can be conventional or part or all of the hardware and/or software can be customized (e.g., optimized) for implementing part or all of the functionality of system 100 described herein.

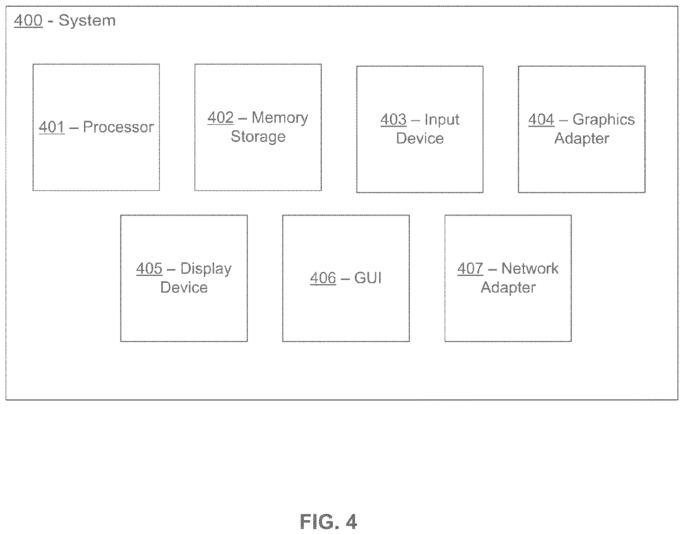

System 100 can include videoconference server 101, health detection server 102, and/or participant devices 103, 104. Videoconference server 101, health detection server 102, and/or participant devices 103, 104 can each be a computer system, such as computer system 400 (FIG. 4), as described below, and can each be a single computer, a single server, a cluster or collection of computers or servers, or a cloud of computers or servers.

Participant devices 103, 104 can comprise any of the elements described in relation to computer system 400 (FIG. 4). For example, participant devices 103, 104 can be mobile devices. A mobile device can refer to a portable electronic device (e.g., an electronic device easily conveyable by hand by a person of average size) with the capability to present audio and/or visual data (e.g., text, images, videos, music, etc.). For example, a mobile electronic device can comprise at least one of a digital media player, a cellular telephone (e.g., a smartphone), a personal digital assistant, a handheld digital computer device (e.g., a tablet personal computer device), a laptop computer device (e.g., a notebook computer device, a netbook computer device), a wearable user computer device, or another portable computer device with the capability to present audio and/or visual data (e.g., images, videos, music, etc.). Thus, in many examples, a mobile electronic device can comprise a volume and/or weight sufficiently small as to permit the mobile electronic device to be easily conveyable by hand.

Exemplary mobile electronic devices can comprise (i) an iPod^{®}, iPhone^{®}, iTouch^{®}, iPad^{®}, MacBook^{®} or similar product by Apple Inc. of Cupertino, California, United States of America, (ii) a Pixel^{™} product or a similar product by Google Inc. of Menlo Park, California, United States of America, (iii) a Lumia^{®} or similar product by the Nokia Corporation of Keilaniemi, Espoo, Finland, and/or (iv) a Galaxy^{™} or similar product by the Samsung Group of Samsung Town, Seoul, South Korea. Further, in the same or different embodiments, a mobile electronic device can comprise an electronic device configured to implement one or more of (i) the iPhone^{®} operating system by Apple Inc. of Cupertino, California, United States of America, (ii) the Android^{™} operating system Google Inc. of Menlo Park, California, United States of America, (iii) the Palm^{®} operating system by Palm, Inc. of Sunnyvale, California, United States, (iv) the Android^{™} operating system developed by the Open Handset Alliance, (v) the Windows Mobile^{™} operating system by Microsoft Corp. of Redmond, Washington, United States of America, or (vi) the Symbian^{™} operating system by Nokia Corp. of Keilaniemi, Espoo, Finland.

Further still, the term "wearable user computer device" or "wearable device" as used herein can refer to an electronic device with the capability to present audio and/or visual data (e.g., text, images, videos, music, etc.) that is configured to be worn by a user and/or mountable (e.g., fixed) on the user of the wearable user computer device (e.g., sometimes under or over clothing; and/or sometimes integrated with and/or as clothing and/or another accessory, such as, for example, a hat, eyeglasses, a wrist watch, shoes, etc.). A wearable user computer device can comprise a mobile electronic device, and vice versa. However, a wearable user computer device does not necessarily comprise a mobile electronic device, and vice versa.

In specific examples, a wearable user computer device can comprise a head mountable wearable user computer device (e.g., one or more head mountable displays, one or more eyeglasses, one or more contact lenses, one or more retinal displays, etc.) or a limb mountable wearable user computer device (e.g., a smart watch, smart ring, etc.). In these examples, a head mountable wearable user computer device can be mountable in close proximity to one or both eyes of a user of the head mountable wearable user computer device and/or vectored in alignment with a field of view of the user.

In more specific examples, a head mountable wearable user computer device can comprise (i) Google Glass^{™} product or a similar product by Google Inc. of Menlo Park, California, United States of America; (ii) the Eye Tap^{™} product, the Laser Eye Tap^{™} product, or a similar product by ePI Lab of Toronto, Ontario, Canada; (iii) Apple Vision Pro^{™} product or similar product by Apple Inc. of Cupertino, California, United States of America; and/or (iv) the Raptyr^{™} product, the STAR 1200^{™} product, the Vuzix Smart Glasses M100^{™} product, or a similar product by Vuzix Corporation of Rochester, New York, United States of America. In other specific examples, a head mountable wearable user computer device can comprise the Virtual Retinal Display^{™} product, or similar product by the University of Washington of Seattle, Washington, United States of America. Meanwhile, in further specific examples, a limb mountable wearable user computer device can comprise the iWatch^{™} product, or similar product by Apple Inc. of Cupertino, California, United States of America, the Galaxy Gear or similar product of Samsung Group of Samsung Town, Seoul, South Korea, the Moto 360 product or similar product of Motorola of Schaumburg, Illinois, United States of America, and/or the Zip^{™} product, One^{™} product, Flex^{™} product, Charge^{™} product, Surge^{™} product, or similar product by Fitbit Inc. of San Francisco, California, United States of America.

Videoconference server 101, health detection server 102, and/or one or more of participant devices 103, 104 can each comprise one or more input devices (e.g., one or more keyboards, one or more keypads, one or more pointing devices such as a computer mouse or computer mice, one or more touchscreen displays, a microphone, etc.), and/or can each comprise one or more display devices (e.g., one or more monitors, one or more touch screen displays, projectors, etc.). In these or other embodiments, one or more of the input device(s) can be similar or identical to input device 403 (FIG. 4). Further, one or more of the display device(s) can be similar or identical to display device 405 (FIG. 4). The input device(s) and the display device(s) can be coupled to the processing module(s) and/or the memory storage module(s) of videoconference server 101, health detection server 102, and/or one or more of participant devices 103, 104 in a wired manner and/or a wireless manner, and the coupling can be direct and/or indirect, as well as locally and/or remotely. As an example of an indirect manner (which may or may not also be a remote manner), a keyboard-video-mouse (KVM) switch can be used to couple the input device(s) and the display device(s) to the processing module(s) and/or the memory storage module(s). In some embodiments, the KVM switch also can be part of videoconference server 101, health detection server 102, and/or one or more of participant devices 103, 104. In a similar manner, the processing module(s) and the memory storage module(s) can be local and/or remote to each other.

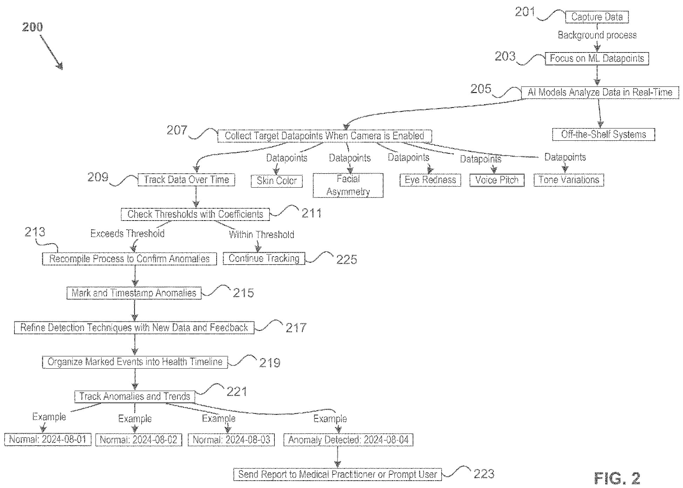

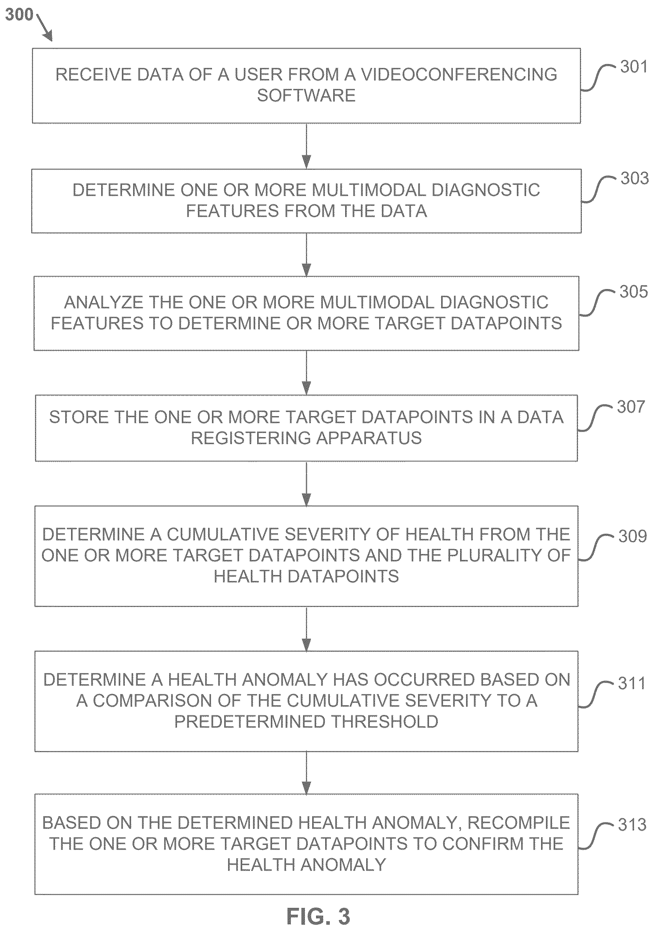

Videoconference server 101 can host and/or run one or more videoconference software platforms. Health detection server 102 can host a system for detecting a user's health status as described herein. For example, health detection server 102 can perform one or more steps of method 200 (FIG. 2) and/or method 300 (FIG. 3). In some embodiments, health detection server 102 can be embodied in and/or distribute a software application capable of performing one or more steps of method 200 (FIG. 2) and/or method 300 (FIG. 3). The software application can be installed/installable on one or more of participant devices 103, 104.

Videoconference server 101, health detection server 102, and/or participant devices 103, 104 can communicate or interface (e.g., interact) with one another through network 120. Network 120 can be an intranet that is not open to the public, a mesh network of individual systems, and/or a distributed system. Accordingly, in many embodiments, videoconference server 101 and/or health detection server 102 (and/or the software used by such systems) can refer to a back end of system 100 operated by an operator and/or administrator of system 100, and participant devices 103, 104 (and/or the software used by such systems) can refer to a front end of system 100 used by one or more participants, respectively. An operator and/or administrator of system 100 can manage system 100, the processing module(s) of system 100, and/or the memory storage module(s) of system 100 using the input device(s) and/or display device(s) of system 100.

Videoconference server 101, health detection server 102, and/or participant devices 103, 104 also can be configured to communicate with one or more databases. The one or more databases can comprise a historical videoconference database that stores records about past videoconferences. A historical videoconference database can also comprise an interaction database containing information about interactions of participant devices with a videoconference. These interactions can be tied to a unique identifier (e.g., an IP address, an advertising ID, device ID, etc.) and/or a user account. In embodiments where a participant interacts with a videoconference before logging into a user account, data stored in the one or more database that is associated with a unique identifier can be merged with and/or associated with data associated with the user account. Data can be deleted from a database when it becomes older than a maximum age, which can be set by an administrator of system 100. Data collected in real-time can be streamed to a database for storage, thereby increasing a storage speed of a database.

The one or more databases can be stored on one or more memory storage modules (e.g., non-transitory memory storage module(s)), which can be similar or identical to the one or more memory storage module(s) (e.g., non-transitory memory storage module(s)) described below with respect to computer system 400 (FIG. 4). Further, the one or more databases can each be stored on a single memory storage module of the memory storage module(s), and/or the non-transitory memory storage module(s) storing the one or more databases or the contents of that particular database can be spread across multiple ones of the memory storage module(s) and/or non-transitory memory storage module(s) storing the one or more databases, depending on the size of the particular database and/or the storage capacity of the memory storage module(s) and/or non-transitory memory storage module(s). In various embodiments, databases can be stored in a cache (e.g., MegaCache) for immediate retrieval on-demand. The one or more databases can each comprise a structured (e.g., indexed) collection of data and can be managed by any suitable database management systems configured to define, create, query, organize, update, and manage database(s). Exemplary database management systems can include MySQL (Structured Query Language) Database, PostgreSQL Database, Microsoft SQL Server Database, Oracle Database, SAP (Systems, Applications, & Products) Database, IBM DB2 Database, and/or NoSQL Database.

Meanwhile, communication between videoconference server 101, health detection server 102, participant devices 103, 104, and/or the one or more databases can be implemented using any suitable manner of wired and/or wireless communication. Accordingly, system 100 can comprise any software and/or hardware components configured to implement the wired and/or wireless communication. Further, the wired and/or wireless communication can be implemented using any one or any combination of wired and/or wireless communication network topologies (e.g., ring, line, tree, bus, mesh, star, daisy chain, hybrid, etc.) and/or protocols (e.g., personal area network (PAN) protocol(s), local area network (LAN) protocol(s), wide area network (WAN) protocol(s), cellular network protocol(s), powerline network protocol(s), etc.). Exemplary PAN protocol(s) can comprise Bluetooth, Zigbee, Wireless Universal Serial Bus (USB), Z-Wave, etc.; exemplary LAN and/or WAN protocol(s) can comprise Institute of Electrical and Electronics Engineers (IEEE) 802.3 (also known as Ethernet), IEEE 802.11 (also known as WiFi), etc.; and exemplary wireless cellular network protocol(s) can comprise Global System for Mobile Communications (GSM), General Packet Radio Service (GPRS), Code Division Multiple Access (CDMA), Evolution-Data Optimized (EV-DO), Enhanced Data Rates for GSM Evolution (EDGE), Universal Mobile Telecommunications System (UMTS), Digital Enhanced Cordless Telecommunications (DECT), Digital Advanced Mobile Phone System (AMPS) (IS-136/Time Division Multiple Access (TDMA)), Integrated Digital Enhanced Network (iDEN), Evolved High-Speed Packet Access (HSPA+), Long-Term Evolution (LTE), WiMAX, etc. The specific communication software and/or hardware implemented can depend on the network topologies and/or protocols implemented, and vice versa. In many embodiments, exemplary communication hardware can comprise wired communication hardware including, for example, one or more data buses, such as, for example, universal serial bus(es), one or more networking cables, such as, for example, coaxial cable(s), optical fiber cable(s), and/or twisted pair cable(s), any other suitable data cable, etc. Further exemplary communication hardware can comprise wireless communication hardware including, for example, one or more radio transceivers, one or more infrared transceivers, etc. Additional exemplary communication hardware can comprise one or more networking components (e.g., modulator-demodulator components, gateway components, etc.).

FIG. 2 is a block diagram of a method 200 for detecting health anomalies from videoconferencing data of a user according to aspects of this disclosure. The method 200 may be initiated from the commencement of use of a videoconferencing platform by a user. For example, the method 200 may be initiated by the beginning of a video call, teleconference, virtual meeting, and the like. In other embodiments, the method 200 may be initiated ad-hoc by a user, such as by the user turning on a setting within the videoconferencing platform. However, this is not meant to be limiting or required as other methods of initiating method 200 may also be possible. For instance, the method 200 may be run as a background process while the machine operating the videoconferencing platform is in use.

Once the method 200 is initiated, the method 200 can begin at block 201 where the system may capture data during video or audio conferences as a background process. The data capture may occur during the normal operation of videoconferencing software (e.g., the data capture may be performed in the background without any action or initiation by the user). The background process may capture and analyze data during video or audio conferences, such as video and/or audio streaming data or communication platform metadata, with minimal user intervention. This can allow the system to gather health-related datapoints. Examples of health-related datapoints may include eye data for diabetic retinopathy detection, skin lesion analysis for melanoma, cardiac function assessment, among others. Therefore, the background processes can covertly monitor health indicators in order to timely detect and report potential health concerns.

The system may capture the data using one or more sensors, such as a camera, microphone, webcam, laptop, keyboard, mouse, or wearable device. As an example, the system may capture audio data, such as sound recordings of a call using the videoconferencing software, or visual data, such as video recordings of a call using the videoconferencing software. The system may also capture data from the keyboard, mouse, or wearable device.

At block 203, the system may focus on machine learning (ML) datapoints that can be used in medical diagnosis. The datapoints that can be used in medical diagnosis can be a datapoint that might indicate a health anomaly. For example, these datapoints may include facial expressions, voice changes, eye movements, etc. where a deviation may be evidence of a health anomaly.

At block 205, the system can use AI models to analyze the data in real-time. The AI models may include facial recognition models, movement tracking models, computer vision models, neural network models, and/or natural language processing models. In some embodiments, the AI models may be off-the-shelf systems that can perform facial recognition and movement tracking, real-time analysis of health indicators, and/or detailed image and video analysis. This architecture of integrating videoconferencing capabilities and medical examination can allow for continuous monitoring but also may leverage AI's capability to learn and adapt over time. More specifically, as the AI models are exposed to more video conferencing data, their accuracy in detecting specific health conditions can improve, making the system more reliable and effective. The method can operate "under the hood," providing health monitoring without disrupting the primary function of video conferencing or requiring any extra effort from the user.

At block 207, the system can collect target datapoints when the camera is enabled. The target datapoints may be collected in a data registering apparatus, such as in the one or more databases described above with respect to FIG. 1. In some embodiments, the system can also collect target datapoints when the camera is not enabled. For example, the system can collect audio data of the call or videoconference metadata (e.g., join/leave events, chat, etc.).

The target datapoints may include skin color, facial asymmetry, eye redness, voice pitch, and/or tone variations. For example, there may be a first tracked health parameter (e.g., eye redness), which may be a list that contains multiple readings of a first particular health parameter collected over time. A second tracked health parameter (e.g., eye or skin color) may be a list that contains multiple readings of a second particular health parameter collected over time. A third tracked health parameter (e.g., voice pitch) may be a list that contains multiple readings of a third particular health parameter collected over time.

However, this is not meant to be limiting or required, as other datapoints may also be collected. Other datapoints may include tardiness or attendance irregularities, escalation events (e.g., raised voices, snapping, or interruptions), stress markers (e.g., heavy breathing, sighing, or visible tension), profanity or aggressive language usage, user outbursts, sentiment and tone deviations (e.g., user is mad always, frustration, sarcasm, or cheerfulness), cognitive delays or processing anomalies (e.g., slow response times; no response from user), behavioral engagement shifts (e.g., user is zoning out or overactive; fixations), physical discomfort indicators (e.g., body language shows discomfort; frequent shifting or sweating), unusual vocal characteristics (e.g., monotony or pitch irregularities; clearing throat), and/or appearance-related anomalies (e.g., disheveled or unkempt; same attire worn for a year).

At block 209, the data may be tracked over time to identify trends and gradual changes in health. For example, the data may be tracked over the courses of a single video call or multiple video calls with a particular user. The tracked health parameters over time, as described above, may be summed or averaged to determine a health indicator value. The average may be calculated by taking a cumulative severity of health parameters over a period of the measured health parameters.

As examples, for eye redness, each collected value may represent a measurement of eye redness, where 0 indicates no redness and 1 indicates maximum redness. In this case, possible values may include, for example, 0.1, 0.2, 0.3, 0.4, 0.5, etc. As another example, for skin color, each value may represent a measurement of skin color deviation, where 1 indicates a significant deviation from the norm. The system can then average the tracked values (e.g., 0.2 + 0.5 + 0.1 + 0.3 + 0.4 = 1.5 / 5 = 0.3) to get the cumulative severity of health indicator value.

For voice pitch, each value may represent the frequency of the voice pitch in Hertz (e.g., 195Hz, 200Hz, 205Hz, 210Hz, etc.). The system can similarity average the tracked values in Hertz (e.g., 200 + 210 + 195 + 205 + 200 = 1010Hz / 5 = 202Hz) to get the cumulative severity of health indicator value.

At block 211, each health indicator value (e.g., cumulative average of values) may be measured against a threshold value. The threshold may be represented by a coefficient. The threshold value may be pre-determined and may be based on medical research for each health parameter. This measurement can determine if the cumulative reading surpasses a medically significant level.

If the health indicator value exceeds these thresholds, at block 213, the system may recompile the process to confirm anomalies. For example, the system may perform the steps described above a second time to confirm that there is in fact an anomaly.

At block 215, the system can mark and timestamp any identified health anomalies. For example, the system can store a flag indicating whether an anomaly is detected for each parameter. The system can also store a timestamp when the anomaly is detected for each parameter. The system may further record instances where one of the health parameters is abnormally high, which can potentially indicate a medical condition.

At block 217, the system may continuously refine detection techniques with new data and expert feedback. For example, the system continuously can receive new data from videoconferences and/or data from experts for identifying health anomalies. The system may be able to train the AI models it uses on this new data to improve the predictions of health anomalies.

At block 219, the system can organize marked events into a health timeline. The health timeline may show a series of tracked values organized by date and time, for example: Normal: 2024-08-01; Normal: 2024-08-02; Normal: 2024-08-03; Anomaly detected: 2024-08-04.

At block 221, the system can use the health timeline to track detected anomalies and trends. For example, the health timeline can help to visualize deviations of health data from the norm or trends to identify the anomalies. Furthermore, by compiling a comprehensive timeline of health data, the system can assist medical professionals with assessing long-term trends and make informed decisions.

At block 223, when the system detects an anomaly, it can automatically send the finding to an external device, such as to a pre-registered medical practitioner, or prompt the user to send the finding to their medical professional.

If the data points do not exceed these thresholds at block 211, the system may continue tracking the data at block 225.

FIG. 3 illustrates a flow diagram of a method 300 for detecting health anomalies from videoconferencing data of a user. The method 300 may be initiated from the commencement of use of a videoconferencing platform by a user. For example, the method 300 may be initiated by the beginning of a video call, teleconference, virtual meeting, and the like. In other embodiments, the method 300 may be initiated ad-hoc by a user, such as by the user turning on a setting within the videoconferencing platform. However, this is not meant to be limiting or required as other methods of initiating method 300 may also be possible. For instance, the method 300 may be run as a background process while the machine operating the videoconferencing platform is in use.

At block 301, the system may receive data of a user from a videoconferencing software. The data may include audio or visual data that is detected by the videoconferencing software while the user in in a virtual meeting. The system may receive the audio data or visual data from one or more sensors used by the videoconferencing software. The sensors may include a plurality of input devices, such as camera, microphone, webcam, laptop, keyboard, mouse, or wearable device. The sensors may be configured to capture detailed audio data or visual data of the user including eye movement, facial expressions, skin color, eye color, and voice tone of the user. The data may also include metadata related to the virtual meeting, such as join/leave events, virtual meeting chat, and the like.

At block 303, the system may determine one or more multimodal diagnostic features from the data. The multimodal diagnostic features may include tardiness or attendance irregularities, escalation events (e.g., raised voices, snapping, or interruptions), stress markers (e.g., heavy breathing, sighing, or visible tension), profanity or aggressive language usage, user outbursts, sentiment and tone deviations (e.g., user is mad always, frustration, sarcasm, or cheerfulness), cognitive delays or processing anomalies (e.g., slow response times; no response from user), behavioral engagement shifts (e.g., user is zoning out or overactive; fixations), physical discomfort indicators (e.g., body language shows discomfort; frequent shifting or sweating), unusual vocal characteristics (e.g., monotony or pitch irregularities; clearing throat), and/or appearance-related anomalies (e.g., disheveled or unkempt; same attire worn for a year).

At block 305, the system may analyze the one or more multimodal diagnostic features to determine one or more target datapoints. The one or more target datapoints may include skin color, facial asymmetry, eye redness, voice pitch, or tone variation. The system may analyze the features using various types of machine learning models. For example, the machine learning models may be a facial recognition and movement tracking model, a real-time analysis of health indicator model, or a detailed image and video analysis model. In some embodiments, the system may apply off-the-shelf models, such as OpenCV to process images to capture and analyze facial expressions and eye movements. In embodiments, the system may also use a monitoring module to track user engagement through metrics such as typing speed and mouse movements, which can facilitate continuous assessment of user responsiveness. In embodiments, the system may analyze behavioral data such as examining eye movements, facial expressions, and voice tones to evaluate cognitive and sensory states in real-time. The system may use a computer vision model to analyze facial expressions in images from the audio data or visual data. Alternatively, or in addition, the system may use a voice analysis model to track tone, pitch, or speech rates of speech from the audio data. The system may also use a face detection model to analyze eye movements in images from the visual data. The system may further use AI to identify discomfort or distraction in users based on the data, which may indicate a cognitive impairment. The discomfort or distraction may include focus deficit, memory challenge, sensory overload, communication difficulty, auditory challenge, poor vision, cognitive overload, limited mobility, or control precision. However, this is not meant to be limiting or required. In other embodiments, the system can analyze the data to assess the state of the user using a computerized algorithm and without the use of AI.

At block 307, the system may store the one or more target datapoints in a data registering apparatus. The data registering apparatus may include a plurality of health datapoints for the user stored over time. The data registering apparatus may be one of the one or more databases as described in FIG. 1.

At block 309, the system can determine a cumulative severity of health from the one or more target datapoints and the plurality of health datapoints. For example, the system can identify that the user is experiencing a health anomaly when the current data deviates from the historical data. In embodiments, the system may average the target datapoints to identify an outlier. In this way, the system can identify behavior of the user or physiological changes that is atypical compared to historical behavior or physiology. Comparing to historical data may also reduce erroneous identification of a health anomaly by comparing to historical behavior or physiology of the specific user. As an example, on a given day analyzed in isolation, a user's eye color or skin color may not indicate an anomaly. However, when compared to historical data, the system may identify a change in eye or skin color that may indicate a vision impairment or onset of dementia.

At block 311, the system can determine a health anomaly has occurred based on a comparison of the cumulative severity to a predetermined threshold. The threshold may be a coefficient, where the cumulative severity being above or below the thresholds may indicate a health condition.

At block 313, the system may, based on the determined health anomaly, recompile the one or more target datapoints to confirm the health anomaly. After confirming the health anomaly, the system may mark and/or flag the health anomaly with an anomaly name and timestamp. With the marked health anomalies, the system can create a health timeline of the marked health anomaly and the plurality of health datapoints from the data registering apparatus. In addition, the system can send the marked health anomaly to an external device. For example, the system may send the marked health anomaly to a pre-registered medical practitioner device. The system may also prompt the user via a user device to send the marked health anomaly to a medical professional.

FIG. 4 illustrates a block diagram of a system 400 that can be employed for detecting health anomalies from videoconferencing data of a user, as described in greater detail below. System 400 is merely exemplary and embodiments of the system are not limited to the embodiments presented herein. System 400 can be employed in many different embodiments or examples not specifically depicted or described herein. In some embodiments, certain elements or modules of system 400 can perform various procedures, processes, and/or activities. In these or other embodiments, the procedures, processes, and/or activities can be performed by other suitable elements or modules of system 400.

Generally speaking, system 400 can be implemented with hardware and/or software. Part or all of the hardware and/or software implemented in system 400 can be conventional or part or all of the hardware and/or software can be customized (e.g., optimized) for implementing part or all of the functionality of system 400 described herein. When implemented as software, one or more elements of system 400 can be emulated (e.g., reproduced functionally and/or by action via software). For example, a virtual machine having one or more elements described below can be instantiated on one or more elements of system 100 (FIG. 1).

When implemented as hardware, one or more of the elements of system 400 can be coupled together using one or more chassis configured to hold one or more circuit boards and/or serial bus(es). These boards and buses allow the various elements of system 400 to communicate amongst each other to accomplish their intended purposes. While elements of system 400 are described below individually, each can also be integrated into one or more chassis, circuit boards, and/or buses of system 400. On the other hand, one or more elements of system 400 can also be removable (e.g., via a PCI slot on a motherboard and/or a USB port). One or more elements of system 400 may also be integrated and/or embedded in a different machine or manufacture. Although specific constructions of boards and buses within system 400 are not shown, it should be understood that their construction can be tied to a form factor selected for system 400.

System 400 can take a number of different form factors based on its implementation. For example, system 400 can be implemented as a desktop computer, a laptop computer, a mobile device, and/or a wearable device as described herein. Further, system 400 can comprise a single computer, a single server, a cluster or collection of computers or servers, or a cloud of computers or servers. Typically, a cluster or collection of servers can be used when the demand on 400 exceeds the reasonable capability of a single server or computer, when a distributed structure for system 400 is desired, and/or when parallel computing is desired.

In many embodiments, system 400 can comprise a processor 401, a memory storage 402, an input device 403, a graphics adapter 404, a display device 405, a graphical user interface (GUI) 406, and/or a network adapter 407.

Generally speaking, processor 401 can comprise any type of computational circuit. For example, processor 401 can comprise a microprocessor, a microcontroller, a controller, a complex instruction set computing (CISC) microprocessor, a reduced instruction set computing (RISC) microprocessor, a very long instruction word (VLIW) microprocessor, a graphics processor, a digital signal processor, application specific integrated circuits (ASICs), etc. Processor 401 can be configured to implement (e.g., run) computer instructions (e.g., program instructions) stored on memory devices in system 400. At least a portion of the program instructions, stored on these devices, can be suitable for carrying out at least part of the techniques and methods described herein. Architecture and/or design of processor 401 can be compliant with any of a variety of commercially distributed architecture families. For example, a processor can have a 32-bit (x86) architecture and/or a 64-bit (x86-64, IA64, and AMD64) architecture. Processor 401 can be configured to perform parallel computing in combination with other elements of system 400 and/or additional processors. Generally speaking, parallel computing can be seen as a technique where multiple elements of system 400 are used to perform calculations simultaneously. In this way, complex and repetitive tasks (e.g., training a predictive software application) can be performed faster and with less processing power than without parallel computing.

Generally speaking, memory storage 402 can comprise non-volatile memory (e.g., read only memory (ROM)) and/or volatile memory (e.g., random access memory (RAM)). The non-volatile memory can be removable and/or non-removable non-volatile memory. Meanwhile, RAM can comprise dynamic RAM (DRAM), static RAM (SRAM), or some other type of RAM. Further, ROM can include mask-programmed ROM, programmable ROM (PROM), one-time programmable ROM (OTP), erasable programmable read-only memory (EPROM), electrically erasable programmable ROM (EEPROM) (e.g., electrically alterable ROM (EAROM) and/or flash memory), or some other type of ROM. Memory storage 402 can comprise non-transitory memory and/or transitory memory. All or a portion of memory storage 402 can be referred to as memory storage module(s) and/or memory storage device(s). Memory storage 402 can have a number of form factors when used in system 400. For example, memory storage 402 can comprise a magnetic disk hard drive, a solid state hard drive, a removable USB storage drive, a RAM chip, etc.

Memory storage 402 can be encoded with a wide variety of computer code configured to operate system 400. For example, portions of memory storage 402 can be encoded with a boot code sequence suitable for restoring system 400 to a functional state after a system reset. As another example, portions of memory storage 402 can comprise microcode such as a Basic Input-Output System (BIOS) operable with elements of system 400. Further, portions of the memory storage 402 can comprise an operating system (e.g., a software program that manages the hardware and software resources of a computer and/or a computer network). The BIOS can be configured to initialize and test components of system 400 and load the operating system. Meanwhile, the operating system can perform basic tasks such as, for example, controlling and allocating memory, prioritizing the processing of instructions, controlling input and output devices, facilitating networking, and/or managing files. Exemplary operating systems can comprise software within the Microsoft^{®} Windows^{®}, Mac OS^{®}, Apple^{®} iOS^{®}, Google^{®} Android^{®}, UNIX^{®}, and/or Linux^{®} series of operating systems.

Input device 403 can be configured to allow a user to interact and/or control elements of system 400. A number of devices can be used as input device 403 alone or in combination. For example, input device 403 can comprise a keyboard, a mouse, a touch screen, a microphone, a camera, etc. Input device 403 can be coupled to other elements of system 400 in a number of ways. For example, input device 403 can be coupled via a Universal Serial Bus (USB) port in a wired and/or wireless manner or via a specialized port (e.g., a PS/2 port) depending on the specific device. User inputs through input device 403 can come in a number of forms. For example, when input device 403 comprises a microphone, user input can be received via voice commands and/or a speech to text software application. As another example, when input device 403 comprises a camera, user input can be received via bodily movements that are captured and interpreted by system 400.

Generally speaking, graphics adapter 404 can be configured to receive and/or generate one or more elements for display on display device 405. Exemplary embodiments of graphics adapter 404 can comprise devices within the NVIDIA^{®} GeForce^{®} and/or the AMD^{®} RX^{®} series of video cards. In many embodiments, a chipset present on graphics adapter 404 can be configured to perform similar, simultaneous computations in a manner more efficient than other chipsets. For example, rendering a 3D scene on graphics adapter 404 can involve repeated geometric calculations performed in parallel to generate the 3D scene. As another example, repeated mathematical calculations involved in training a predictive software application can be performed in parallel on graphics adapter 404 more efficiently thank on processor 401. Display device 405 can receive and display signals from graphics adapter 404. A number of devices can be used as display device 405. For example, display device 405 can comprise a computer monitor, a television, a touch screen display, a heads up display (HUD) medium, etc.

In some embodiments, display device 405 can optionally display graphical user interface (GUI) 406. GUI 406 can be a part of and/or displayed by participant devices 103, 104. With regards to form, GUI 406 can comprise text and/or graphics (image) based user interfaces. For example, GUI 406 can comprise a heads up display (HUD). When GUI 406 comprises a HUD, GUI 406 can be projected onto a medium (e.g., glass, plastic, metal, etc.), displayed in midair as a hologram, and/or displayed on display device 405. GUI 406 can be color, black and white, and/or greyscale. GUI 406 can be implemented as an application running on a computer system, such as computer system 400, videoconference server 101 (FIG. 1), health detection server 102 (FIG. 1), and/or participant devices 103, 104 (FIG. 1). GUI 406 can also comprise a website accessed through a network (e.g., network 120 (FIG. 1)). For example, GUI 406 can comprise a cloud storage website. When GUI 406 allows for modification and/or changes to one or more settings in system 400, it can be referred to as an administrative (e.g., back end) GUI. GUI 406 can also be displayed as or on a virtual reality (VR) and/or augmented reality (AR) system or display. GUI 406 can receive a number of interactions from a user via input device 403. For example, an interaction with a GUI can comprise a click, a look, a selection, a grab, a view, a purchase, a bid, a swipe, a pinch, a reverse pinch, etc.

Network adapter 407 can be configured to connect system 400 to a computer network by wired communication (e.g., a wired network adapter) and/or wireless communication (e.g., a wireless network adapter). Network adapter 407 can be integrated into one or more chassis, circuit boards, and/or buses or be removable (e.g., via a PCI slot on a motherboard). For example, network adapter 407 can be implemented via one or more dedicated communication chips configured to receive various protocols of wired and/or wireless communications.

For simplicity and clarity of illustration, the drawing figures illustrate the general manner of construction, and descriptions and details of some features and techniques may be omitted to avoid unnecessarily obscuring the present disclosure. Additionally, elements in the drawing figures are not necessarily drawn to scale. For example, the dimensions of some of the elements in the figures may be exaggerated relative to other elements to help improve understanding of embodiments of the present disclosure. The same reference numerals in different figures denote the same elements.

The terms "first," "second," "third," "fourth," and the like in the description and in the claims, if any, are used for distinguishing between similar elements and not necessarily for describing a particular sequential or chronological order. It is to be understood that the terms so used are interchangeable under appropriate circumstances such that the embodiments described herein are, for example, capable of operation in sequences other than those illustrated or otherwise described herein. Furthermore, the terms "include," and "have," and any variations thereof, are intended to cover a non-exclusive inclusion, such that a process, method, system, article, device, or apparatus that comprises a list of elements is not necessarily limited to those elements but may include other elements not expressly listed or inherent to such process, method, system, article, device, or apparatus.

The terms "left," "right," "front," "back," "top," "bottom," "over," "under," and the like in the description and in the claims, if any, are used for descriptive purposes and not necessarily for describing permanent relative positions. It is to be understood that the terms so used are interchangeable under appropriate circumstances such that the embodiments of the apparatus, methods, and/or articles of manufacture described herein are, for example, capable of operation in other orientations than those illustrated or otherwise described herein.

The terms "couple," "coupled," "couples," "coupling," and the like should be broadly understood and refer to connecting two or more elements mechanically and/or otherwise. Two or more electrical elements may be electrically coupled together, but not be mechanically or otherwise coupled together. Coupling may be for any length of time, e.g., permanent or semi-permanent or only for an instant. "Electrical coupling" and the like should be broadly understood and include electrical coupling of all types. The absence of the word "removably," "removable," and the like near the word "coupled," and the like does not mean that the coupling, etc. in question is or is not removable.

As defined herein, two or more elements are "integral" if they are comprised of the same piece of material. As defined herein, two or more elements are "non-integral" if each is comprised of a different piece of material.

As defined herein, "real-time" can, in some embodiments, be defined with respect to operations carried out as soon as practically possible upon occurrence of a triggering event. A triggering event can include receipt of data necessary to execute a task or to otherwise process information. Because of delays inherent in transmission and/or in computing speeds, the term "real time" encompasses operations that occur in "near" real time or somewhat delayed from a triggering event. In a number of embodiments, "real time" can mean real time less a time delay for processing (e.g., determining) and/or transmitting data. The particular time delay can vary depending on the type and/or amount of the data, the processing speeds of the hardware, the transmission capability of the communication hardware, the transmission distance, etc. However, in many embodiments, the time delay can be less than approximately one second, two seconds, five seconds, or ten seconds.

As defined herein, "approximately" can, in some embodiments, mean within plus or minus ten percent of the stated value. In other embodiments, "approximately" can mean within plus or minus five percent of the stated value. In further embodiments, "approximately" can mean within plus or minus three percent of the stated value. In yet other embodiments, "approximately" can mean within plus or minus one percent of the stated value.
Although systems and methods for context dependent invocation of predictive software application and data storage have been described with reference to specific embodiments, it will be understood by those skilled in the art that various changes may be made without departing from the scope of the invention, as defined by the claims. For the purpose of determining the extent of protection conferred by the claims, due account shall be taken of any element which is equivalent to an element specified in the claims

## Claims

1. A computerized method for detecting health anomalies from videoconferencing data of a user, the method comprising:
receiving audio data or visual data of the user from a videoconferencing software;
determining one or more multimodal diagnostic features from the audio data or visual data;
analyzing, using a machine learning model, the one or more multimodal diagnostic features to determine one or more target datapoints;
storing the one or more target datapoints in a data registering apparatus, the data registering apparatus comprising a plurality of health datapoints for the user stored over time;
determining a cumulative severity of health from the one or more target datapoints and the plurality of health datapoints;
determining a health anomaly has occurred based on a comparison of the cumulative severity to a predetermined threshold;
based on the determined health anomaly, recompiling the one or more target datapoints to confirm the health anomaly;
marking the health anomaly with an anomaly name and timestamp;
creating a health timeline of the marked health anomaly and the plurality of health datapoints from the data registering apparatus; and
sending the marked health anomaly to an external device.

2. The computerized method of claim 1, wherein the one or more multimodal diagnostic features comprise one or more of: meeting attendance irregularities, voice escalations, aggressive language usage, tone deviations, cognitive delays, behavioral engagement shifts, heavy breathing, shifting in body language, appearance-related anomalies, facial expressions, voice changes, or irregular eye movements.

3. The computerized method of claim 1 or claim 2, wherein the machine learning model comprises one of: a facial recognition and movement tracking model, a real-time analysis of health indicator model, or a detailed image and video analysis model.

4. The computerized method of any preceding claim, wherein the one or more target datapoints comprise one or more of: skin color, facial asymmetry, eye redness, voice pitch, or tone variation.

5. The computerized method of any preceding claim, wherein the threshold is a coefficient.

6. The computerized method of any preceding claim, further comprising refining the determining of the one or more multimodal diagnostic features with new data and expert feedback.

7. The computerized method of any preceding claim, wherein sending the marked health anomaly to the external device comprises sending the marked health anomaly to a pre-registered medical practitioner device.

8. The computerized method of any preceding claim, wherein sending the marked health anomaly to the external device comprises prompting the user via a user device to send the marked health anomaly to a medical professional.

9. A system comprising:
one or more sensors integrated into a videoconferencing software configured to receive audio data or visual data of a user;
a processor configured to:
determine one or more multimodal diagnostic features from the audio data or visual data;
analyze, using a machine learning model, the one or more multimodal diagnostic features to determine one or more target datapoints;
store the one or more target datapoints in a data registering apparatus, the data registering apparatus comprising a plurality of health datapoints for the user stored over time;
determine a cumulative severity of health from the one or more target datapoints and the plurality of health datapoints;
determine a health anomaly has occurred based on a comparison of the cumulative severity to a predetermined threshold;
mark the health anomaly with an anomaly name and timestamp; and
create a health timeline of the marked health anomaly and the plurality of health datapoints from the data registering apparatus.

10. The system of claim 9, wherein the one or more multimodal diagnostic features comprise one or more of: meeting attendance irregularities, voice escalations, aggressive language usage, tone deviations, cognitive delays, behavioral engagement shifts, heavy breathing, shifting in body language, appearance-related anomalies, facial expressions, voice changes, or irregular eye movements.

11. The system of claim 9 or claim 10, wherein the machine learning model comprises one of: a facial recognition and movement tracking model, a real-time analysis of health indicator model, or a detailed image and video analysis model.

12. The system of any of claims 9 to 11, wherein the one or more target datapoints comprise one or more of: skin color, facial asymmetry, eye redness, voice pitch, or tone variation.

13. The system of any of claims 9 to 12, wherein the processor is further configured to send the marked health anomaly to an external device.

14. A computerized method comprising:
receiving videoconferencing data of the user from a videoconferencing software;
determining one or more multimodal diagnostic features from the videoconferencing data;
analyzing the one or more multimodal diagnostic features to determine one or more target datapoints;
storing the one or more target datapoints in a data registering apparatus, the data registering apparatus comprising a plurality of health datapoints for the user stored over time;
determining a cumulative severity of health from the one or more target datapoints and the plurality of health datapoints;
determining a health anomaly has occurred based on a comparison of the cumulative severity to a predetermined threshold;
based on the determined health anomaly, recompiling the one or more target datapoints to confirm the health anomaly; and
sending the health anomaly to an external device.

15. The computerized method of claim 14, wherein the one or more multimodal diagnostic features comprise one or more of: meeting attendance irregularities, voice escalations, aggressive language usage, tone deviations, cognitive delays, behavioral engagement shifts, heavy breathing, shifting in body language, appearance-related anomalies, facial expressions, voice changes, or irregular eye movements,
and/or wherein the one or more target datapoints are determined using a machine learning, and/or wherein the one or more target datapoints comprise one or more of: skin color, facial asymmetry, eye redness, voice pitch, or tone variation,
and/or wherein the threshold is a coefficient,
and/or further comprising refining the determining of the one or more multimodal diagnostic features with new data and expert feedback,
and/or wherein sending the marked health anomaly to the external device comprises sending the marked health anomaly to a pre-registered medical practitioner device.
